Europäisches Patentamt

European Patent Office

Office européen des brevets

(19)

(11) Publication number: **0 121 352**
**B1**

(12) **EUROPEAN PATENT SPECIFICATION**

(45) Date of publication of patent specification: **07.01.88**

(21) Application number: **84301468.9**

(22) Date of filing: **06.03.84**

(51) Int. Cl.⁴: **C 12 N 15/00,** C 12 N 9/38, C 12 N 9/52

(54) **A leader sequence to promote the secretion of gene products.**

(30) Priority: **28.03.83 GB 8308483**

(43) Date of publication of application:
**10.10.84 Bulletin 84/41**

(45) Publication of the grant of the patent:
**07.01.88 Bulletin 88/01**

(84) Designated Contracting States:
**BE CH DE FR GB IT LI NL SE**

(56) References cited:
**EP-A-0 001 931**
**EP-A-0 049 619**

(73) Proprietor: **The Public Health Laboratory Service Board**
**61 Colindale Avenue**
**London NW9 5EQ (GB)**

(72) Inventor: **Atkinson, Anthony**
**Twigley Mill Corner Winterbourne Gunner**
**Salisbury Wiltshire SP 4 6JJ (GB)**
Inventor: **Minton, Nigel Peter**
**6 Highbury Avenue**
**Salisbury Wiltshire SP2 7EX (GB)**
Inventor: **Sherwood, Roger Franklin**
**35 Attwood Road**
**Salisbury Wiltshire SP1 3PR (GB)**

(74) Representative: **James, Stephen Richard et al**
**Procurement Executive Ministry of Defence Patents 1A(4), Room 2014 Empress State Building**
**Lillie Road London SW6 1TR (GB)**

Courier Press, Leamington Spa, England.

EP 0 121 352 B1

## 0 121 352

**Description**

The present invention relates to fragments of specific deoxyribonucleotide sequences that promote the secretion of gene products from cells and in particular to recombinant DNA transfer vectors that contain these fragments.

Recent developments in biochemistry have led to the construction of recombinant DNA transfer vectors in which, transfer vectors, for example plasmids, are made to contain exogeneous DNA. In some cases the recombinant incorporates heterologous DNA that codes for polypeptides that are ordinarily not produced by the organism susceptible to transformation by the recombinant vehicle.

In its basic outline a method of endowing a microorganism with the ability to synthesise a new protein involves three general steps:

(a) isolation and purification of the specific gene or nucleotide sequences containing the genetically coded information for the amino acid sequence of the desired protein or polypeptide,

(b) recombination of the isolated gene or nucleotide sequence with an appropriate transfer vector, typically DNA of a bacteriophage or plasmid to form a recombinant transfer vector that codes, in part, for the production of the desired protein or polypeptide,

(c) transfer of the vector to the appropriate microorganism and selection of a strain of the recipient microorganism containing the desired genetic information.

Provided the gene or nucleotide sequence expresses its protein or polypeptide in the chosen microorganism, growth of the microorganism should then produce the desired protein or polypeptide in significant quantities.

Once the microorganism has been cultured, the protein or polypeptide must be isolated from the undesired materials. This step is considerably facilitated if the majority of the desired protein or polypeptide is present in the culture medium and/or the periplasmic space of the microorganism. In other words purification may be performed in a more efficient manner if, once expressed, the protein or polypeptide passes through the cell membrane and out of the cytoplasm.

The passage of the protein or polypeptide through the cell membrane is desirable for two main reasons. First the desired protein or polypeptide will generally be foreign to the microorganism in which it is expressed. In many cases, therefore, it will be quickly broken down by proteolytic enzymes etc. in the cells cytoplasm and will, subsequently, have a short half life within the cell. By transferring the protein or polypeptide out of the cytoplasm soon after expression the stability of the protein or polypeptide will be greatly increased. Second the number of unwanted genetic materials and products (from which the desired protein or polypeptide must be isolated) will be far greater in the cell's cytoplasm than in the culture medium and/or in the cell's periplasmic space. It can be seen that on both of the above counts the transfer of the protein or polypeptide through the cell membrane and out of the cytoplasm will greatly facilitate protein or polypeptide isolation.

One way in which the secretion of gene products from the cell's cytoplasm may be promoted is to produce, within the cytoplasm, a preprotein or prepolypeptide in which the desired protein or polypeptide is preceded by a signal polypeptide. The predominantly hydrophobic signal polypeptide directs the desired protein or polypeptide to the cell's periplasmic space, where the signal peptide is removed as the desired protein or polypeptide traverses the cell membrane.

Many of the known signal peptides contain cysteine residues. These residues have been found to react in the cell membrane and thereby inhibit the efficient transfer of the desired gene product out of the cell.

It is the primary object of the present invention to provide recombinant DNA transfer vectors containing a leader sequence polynucleotide that codes for a signal peptide that is cysteine free. Other objects and advantages of the present invention will become apparent from the following description thereof.

According to the present invention there is provided a recombinant DNA transfer vector comprising a leader sequence polynucleotide coding for signal polypeptide of formula I,

Met-Arg-Pro-Ser-Ile-His-Arg-Thr-Ala-Ile-Ala-          I
Ala-Val-Leu-Ala-Thr-Ala-Phe-Val-Ala-Gly-Thr

The transfer vector may be a bacteriophage or, which is preferred, a plasmid.

Preferably the majority of the codons in the nucleotide sequence are those preferred for the expression of microbial genomes. Suitable codons are listed in UK 1,568,047 and UK 2007675A, and these publications are therefore incorporated herein by reference.

In one preferred embodiment of the present transfer vector the nucleotide sequence has formula II

| 5'— | ATG | CGC | CCA | TCC | ATC | CAC | CGC | ACA | |
| | GCC | ATC | GCC | GCC | GTG | CTG | GCC | ACC | II |
| | GCC | TTC | GTG | GCG | GGC | ACC | — 3' | | |

The nucleotide sequence coding for the signal polypeptide (the leader sequence polynucleotide) will preferably be downstream of and in reading phase with a bacterial or a yeast promoter and a prokaryotic

2

ribosome binding site in the transfer vector. Moreover the leader sequence polynucleotide will either be upstream of an insertion site for a structural gene or, which is preferred, will be upstream of and in reading phase with a structural gene coding for a desired protein or polypeptide. Preferably the gene codes for a eukaryotic, particularly a mammalian, protein or polypeptide.

The structural gene may code for such prokaryotic proteins as E. coli β-galactosidase or Pseudomonas carboxy peptidase $G_2$ (CPG$_2$) (Carboxypeptidase $G_2$ is an enzyme, produced by Pseudomonas species strain RS-16, that has application in cancer chemotherapy. It is a $Zn^{2+}$ containing dimer of $2 \times 42,000$ daltons and has high affinities (Km values of $10^{-5}$ or $10^{-6}M$) for both 5-methyltetrahydrofolate, the predominant circulatory form of folate in mammals and for the folic acid antagonist methotrexate (MTX), which is widely used in cancer chemotherapy. The enzyme may be used directly for the plasma depletion of reduced folates, essential as co-factors in purine and particularly in pyrimidine biosynthesis. CPG$_2$ has been shown to inhibit the development of the Walker 256 carcinoma *in vivo* and to remove MTX from circulation in patients where prolonged exposure to high doses of MTX leads to toxicity).

Examples of transfer vectors according to the present invention that code for CPG$_2$ are pNM1, pNM111, pNM14, pNM21, pNM22, pNM31, pNM32 and pLEC3.

The promoter is preferably a high expression bacterial or yeast promoter for the structural gene in a variety of hosts. The particular choice of promoter will depend on the microorganism to be transformed. For example the transformation of E. coli will generally be effected by a transfer vector in which an E. coli promoter controls the expression of the structural gene. Examples of E. coli promoters are those present in the plasmids pBR 322 and pAT 153. By contrast, the transformation of Pseudomonas species will generally be effected by a transfer vector in which a Pseudomonas promoter controls the expression of the structural gene. Examples of Pseudomonas promoters are those present in the plasmid pKT 230 or Pseudomonas chromosomal DNA.

In order to express the structural gene the present transfer vector will be transformed into a suitable microorganism. According to a further aspect of the present invention therefore there is provided a microorganism transformed by a recombinant DNA transfer vector according to this invention. The microorganism will preferably be a bacterium or yeast in which high expression of the structural gene, within the transfer vector, occurs. Depending on the choice of promoter the microorganism may be a strain chosen from one of the following bacteria E. coli, Pseudomonas or the yeast Saccharomyces cerevisiae.

Having transformed the microorganism, the protein or polypeptide, for which the structural gene codes, may then be expressed by culturing the transformed microorganism in a culture medium. It is the primary advantage of the present invention that culturing the transformed microorganism affords a preprotein or prepolypeptide in which the desired protein or polypeptide is preceded by the present signal polypeptide. This means that soon after expression the signal polypeptide directs the desired protein or polypeptide to the cell's periplasmic space, where the signal polypeptide is removed as the desired protein or polypeptide traverses the cell membrane. Since the present signal polypeptide is free of cysteine residues the desired gene product will be efficiently secreted through the membrane.

The present transfer vectors may be prepared by any of the methods that are well known in the recombinant DNA art. For example the leader sequence polynucleotide may be synthesised by the modified triester method of K. Itakura et al, *JACS*, 1975, *97*, 7327 or by the improved oligodeoxynucleotide preparation described in UK 2007675A. The disclosure of both of these references is incorporated herein by reference. The synthesised polynucleotide may then be inserted in a transfer vector, preferably a plasmid. In the transfer vector it will preferably be downstream of and in reading phase with a bacterial or a yeast promoter and a prokaryotic ribosome binding site. The leader sequence polynucleotide should also be either upstream of a structural gene insertion site or upstream of and in reading phase with a structural gene.

Alternatively, DNA fragments containing the leader sequence polynucleotide may be obtained from natural sources, in particular from the chromosomal DNA of Pseudomonas species strain RS-16. In this particular case a polynucleotide (formula II above) coding for the present signal polypeptide immediately precedes a structural gene coding for CPG$_2$. A number of the DNA fragments containing this leader sequence polynucleotide may therefore be recognised by their ability, on insertion into a plasmid and transformation of a microorganism by the resultant recombinant vector, to enable a microorganism to grow on folate. Examples of such recombinant transfer vectors that contain both a polynucleotide coding for the present signal polypeptide (formula II above) and a structural gene coding for CPG$_2$ are pNM1, pNM111, pNM14, pNM21, pNM22, pNM31, pNM32 and pLEC3. Of course, once a Fol$^+$ recombinant vector has been obtained in this way it may be subcloned to afford alternative vectors (either Fol$^+$ or Fol$^-$) that also contain a polynucleotide coding for the present signal polypeptide.

Once a suitable DNA fragment has been isolated it may then be inserted in a transfer vector, preferably a plasmid. In the transfer vector the leader sequence polynucleotide on the inserted fragment should be downstream of and in reading phase with a bacterial or a yeast promoter and a prokaryotic ribosome binding site. The leader sequence polynucleotide should also be either upstream of a structural gene insertion site or upstream of and in reading phase with a structural gene.

The structural gene for insertion downstream of and in reading phase with the present leader sequence polynucleotide may be obtained, for example, by the synthetic methods mentioned above (this is particularly useful for the preparation of genes coding for small proteins). Alternatively the structural gene

# 0 121 352

may be prepared from m=RNA by the use of the enzyme reverse transcriptase or may be isolated from natural sources (chromosomal DNA).

An example of the latter method is the isolation of DNA fragments containing a polynucleotide sequence (shown in Table 1) coding for the enzyme $CPG_2$ (amino acid sequence also shown in Table 1) from Pseudomonas species strain RS-16 chromosomal DNA. Examples of plasmids containing a $CPG_2$ structural gene, as well as a polynucleotide coding for the present signal polypeptide (formula II above), are pNM1, pNM111, pNM14, pNM21, pNM22, pNM31, pNM32 and pLEC3.

Once prepared or isolated the leader sequence polynucleotide and the structural gene will be inserted into a transfer vector, preferably a plasmid, to form a recombinant DNA transfer vector according to the present invention. The insertion step or steps will preferably be effected by one of the well known techniques in this art that employ restriction endonucleases, see for example the methods discussed in UK 2090600A, the disclosure of which is incorporated herein by reference. The choice of transfer vector will be determined by the microorganism in which the leader sequence polynucleotide and structural gene are to be expressed. Generally the transfer vector will be a cloning vehicle that is suitable for transforming the chosen microorganisms and that displays a phenotypical characteristic, such as antibiotic resistance, by which the recombinant transfer vectors may be selected. Thus, if the microorganism is to be E. coli, then suitable transfer vectors will be the E. coli plasmids pBR322 and pAT153. Alternatively, if the microorganism is to be Pseudomonas then a suitable transfer vector will be Pseudomonas pkT230.

TABLE 1

A polynucleotide sequence, coding for $CPG_2$, isolated from
Pseudomonas species strain RS-16 chromosomal DNA

| | 1 | | | | | | | |
|---|---|---|---|---|---|---|---|---|
| | Met | Arg | Pro | Ser | Ile | His | Arg | Thr |
| 5'— | ATG | CGC | CCA | TCC | ATC | CAC | CGC | ACA |
| | 10 | | | | | | | |
| Ala | Ile | Ala | Ala | Val | Leu | Ala | Thr | Ala |
| GCC | ATCC | GCC | GCC | GTG | CTG | GCC | ACC | GCC |
| | | 20 | | | | | | |
| Phe | Val | Ala | Gly | Thr | Ala | Leu | Ala | Gln |
| TTC | GTG | GCG | GGC | ACC | GCC | CTG | GCC | CAG |
| | | | 30 | | | | | |
| Lys | Arg | Asp | Asn | Val | Leu | Phe | Gln | Ala |
| AAG | CGC | GAC | AAC | GTG | CTG | TTC | CAG | GCA |
| | | | | 40 | | | | |
| Ala | Thr | Asp | Glu | Gln | Pro | Ala | Val | Ile |
| GCT | ACC | GAC | GAG | CAG | CCG | GCC | GTG | ATC |
| | | | | | 50 | | | |
| Lys | Thr | Leu | Glu | Lys | Leu | Val | Asn | Ile |
| AAG | ACG | CTG | GAG | AAG | CTG | GTC | AAC | ATC |
| | | | | | | 60 | | |
| Glu | Thr | Gly | Thr | Gly | Asp | Ala | Glu | Gly |
| GAG | ACC | GGC | ACC | GGT | GAC | GCC | GAG | GGC |
| | | | | | | | 70 | |
| Ile | Ala | Ala | Ala | Gly | Asn | Phe | Leu | Glu |
| ATC | GCC | GCT | GCG | GGC | AAC | TTC | CTC | GAG |
| | | | | | | | | 80 |
| Ala | Glu | Leu | Lys | Asn | Leu | Gly | Phe | Thr |
| GCC | GAG | CTC | AAG | AAC | CTC | GGC | TTC | ACG |
| Val | Thr | Arg | Ser | Lys | Ser | Ala | Gly | Leu |
| GTC | ACG | CGA | AGC | AAG | TCG | GCC | GGC | CTG |
| 90 | | | | | | | | |
| Val | Val | Gly | Asp | Asn | Ile | Val | Gly | Lys |
| GTG | GTG | GGC | GAC | AAC | ATC | GTG | GGC | AAG |

4

TABLE 1 (contd.)

|  |  | 100 |  |  |  |  |  |  |
|---|---|---|---|---|---|---|---|---|
| Ile | Lys | Gly | Arg | Gly | Gly | Lys | Asn | Leu |
| ATC | AAG | GGC | CGC | GGC | GGC | AAG | AAC | CTG |

|  |  | 110 |  |  |  |  |  |  |
|---|---|---|---|---|---|---|---|---|
| Leu | Leu | Met | Ser | His | Met ᵔ | Asp | Thr | Val |
| CTG | CTG | ATG | TCG | CAC | ATG | GAC | ACC | GTC |

|  |  |  | 120 |  |  |  |  |  |
|---|---|---|---|---|---|---|---|---|
| Tyr | Leu | Lys | Gly | Ile | Leu | Ala | Lys | Ala |
| TAC | CTC | AAG | GGC | ATT | CTC | GCG | AAG | GCC |

|  |  |  |  | 130 |  |  |  |  |
|---|---|---|---|---|---|---|---|---|
| Pro | Phe | Arg | Bal | · Glu | Gly | Asp | Lys | Ala |
| CCG | TTC | CGC | GTC | GAA | GGC | GAC | AAG | GCC |

|  |  |  |  |  | 140 |  |  |  |
|---|---|---|---|---|---|---|---|---|
| Tyr | Gly | Pro | Gly | Ile | Ala | Asp | Asp | Lys |
| TAC | GGC | CCG | GGC | ATC | GCC | GAC | GAC | AAG |

|  |  |  |  |  |  | 150 |  |  |
|---|---|---|---|---|---|---|---|---|
| Gly | Gly | Asn | Ala | Val | Ile | Leu | His | Thr |
| GGC | GGC | AAC | GCG | GTC | ATC | CTG | CAC | ACG |

|  |  |  |  |  |  |  | 160 |  |
|---|---|---|---|---|---|---|---|---|
| Leu | Lys | Leu | Leu | Lys | Glu | Tyr | Gly | Val |
| CTC | AAG | CTG | CTG | AAG | GAA | TAC | GGC | GTG |

|  |  |  |  |  |  |  |  | 170 |
|---|---|---|---|---|---|---|---|---|
| Arg | Asp | Tyr | Gly | Thr | Ile | Thr | Val | Leu |
| CGC | GAC | TAC | GGC | ACC | ATC | ACC | GTG | CTG |

|  |  |  |  |  |  |  |  |  |
|---|---|---|---|---|---|---|---|---|
| Phe | Asn | Thr | Asp | Glu | Glu | Lys | Gly | Ser |
| TTC | AAC | ACC | GAC | GAG | GAA | AAG | GGT | TCC |

| 180 |  |  |  |  |  |  |  |  |
|---|---|---|---|---|---|---|---|---|
| Phe | Gly | Ser | Arg | Asp | Leu | Ile | Gln | Glu |
| TTC | GGC | TCG | CGC | GAC | CTG | ATC | CAG | GAA |

|  | 190 |  |  |  |  |  |  |  |
|---|---|---|---|---|---|---|---|---|
| Glu | Ala | Lys | Leu | Ala | Asp | Tyr | Val | Leu |
| GAA | GCC | AAG | CTG | GCC | GAC | TAC | CTG | CTC |

|  |  | 200 |  |  |  |  |  |  |
|---|---|---|---|---|---|---|---|---|
| Ser | Phe | Glu | Pro | Thr | Ser | Ala | Gly | Asp |
| TCC | TTC | GAG | CCC | ACC | AGC | GCA | GGC | GAC |

|  |  |  | 210 |  |  |  |  |  |
|---|---|---|---|---|---|---|---|---|
| Glu | Lys | Leu | Ser | Leu | Gly | Thr | Ser | Gly |
| GAA | AAA | CTC | TCG | CTG | GGC | ACC | TCG | GGC |

|  |  |  |  | 220 |  |  |  |  |
|---|---|---|---|---|---|---|---|---|
| Ile | Ala | Tyr | Val | Gln | Val | Asn | Ile | Thr |
| ATC | GCC | TAC | GTG | CAG | GTC | AAC | ATC | ACC |

|  |  |  |  |  | 230 |  |  |  |
|---|---|---|---|---|---|---|---|---|
| Gly | Lys | Ala | Ser | His | Ala | Gly | Ala | Ala |
| GGC | AAG | GCC | TCG | CAT | GCC | GGC | GCC | GCG |

|  |  |  |  |  |  | 240 |  |  |
|---|---|---|---|---|---|---|---|---|
| Pro | Glu | Leu | Gly | Val | Asn | Ala | Leu | Val |
| CCC | GAG | CTG | GGC | GTG | AAC | GCG | CTG | GTC |

5

TABLE 1 (contd.)

| | | | | | | | | |
|---|---|---|---|---|---|---|---|---|
| Glu GAG | Ala GCT | Ser TCC | Asp GAC | Leu CTC | Val GTG | Leu CTG | **250** Arg CGC | Thr ACG |
| Met ATG | Asn AAC | Ile ATC | Asp GAC | Asp GAC | Lys AAG | Ala GCG | Lys AAG | **260** Asn AAC |
| Leu CTG | Arg CGC | Phe TTC | Asn AAC | Trp TGG | Thr ACC | Ile ATC | Ala GCC | Lys AAG |
| **270** Ala GCC | Gly GGC | Asn AAC | Val GTC | Ser TCG | Asn AAC | Ile ATC | Ile ATC | Pro CCC |
| Ala GCC | **280** Ser AGC | Ala GCC | Thr ACG | Leu CTG | Asn AAC | Ala GCC | Asp GAC | Val GTG |
| Arg CGC | Tyr TAC | **290** Ala GCG | Arg CGC | Asn AAC | Glu GAG | Asp GAC | Phe TTC | Asp GAC |
| Ala GCC | Ala GCC | Met ATG | **300** Lys AAG | Thr ACG | Leu CTG | Glu GAA | Glu GAG | Arg CGC |
| Ala GCG | Gln CAG | Gln CAG | Lys AAG | **310** Lys AAG | Leu CTG | Pro CCC | Glu GAG | Ala GCC |
| Asp GAC | Val GTG | Lys AAG | Val GTG | Ile ATC | **320** Val GTC | Thr ACG | Arg CGC | Gly GGC |
| Arg CGC | Pro CCG | Ala GCC | Phe TTC | Asn AAT | Ala GCC | **330** Gly GGC | Glu GAA | Gly GGC |
| Gly GGC | Lys AAG | Lys AAG | Leu CTG | Val GTC | Asp GAC | Lys AAG | **340** Ala GCG | Val GTG |
| Ala GCC | Tyr TAC | Tyr TAC | Lys AAG | Glu GAA | Ala GCC | Gly GGC | Gly GGC | **350** Thr ACG |
| Leu CTG | Gly GGC | Val GTG | Glu GAA | Glu GAG | Arg CGC | Thr ACC | Gly GGC | Gly GGC |
| **360** Gly GGC | Thr ACC | Asp GAC | Ala GCG | Ala GCC | Tyr TAC | Ala GCC | Ala GCG | Leu CTC |
| Ser TCA | **370** Gly GGC | Lys AAG | Pro CCA | Val GTG | Ile ATC | Glu GAG | Ser AGC | Leu CTG |
| Gly GGC | Leu CTG | **380** Pro CCG | Gly GGC | Phe TTC | Gly GGC | Tyr TAC | His CAC | Ser AGC |

**0 121 352**

TABLE 1 (contd.)

| | | | 390 | | | | | |
|---|---|---|---|---|---|---|---|---|
| Asp | Lys | Ala | Glu | Tyr | Val | Asp | Ile | Ser |
| GAC | AAG | GCC | GAG | TAC | GTG | GAC | ATC | AGC |

| | | | | 400 | | | | |
|---|---|---|---|---|---|---|---|---|
| Ala | Ile | Pro | Arg | Arg | Leu | Tyr | Met | Ala |
| GCG | ATT | CCG | CGC | CGC | CTG | TAC | ATG | GCT |

| | | | | | 410 | | | |
|---|---|---|---|---|---|---|---|---|
| Ala | Arg | Leu | Ile | Met | Asp | Leu | Gly | Ala |
| CGC | CGC | CTG | ATC | ATG | GAT | CTG | GGC | GCC |

| | | | |
|---|---|---|---|
| Gly | Lys | | |
| GGC | AAG | TGA | —3' |

Amino acids 1 to 22 are the present signal polypeptide.
Amino acids 23 to 415 are the $CPG_2$ structural gene.
NB The leader sequence polynucleotide is the preferred polynucleotide of formula II.

The present recombinant DNA transfer vectors, microorganisms transformed by the present recombinant DNA transfer vectors and processes for the preparation of said vectors and microorganisms will now be described by way of example only, with particular reference to the Figures in which:
Figure 1 is a restriction enzyme cleavage site map of pNM1,
Figure 2 is a restriction enzyme cleavage site map of pNM111,
Figure 3 is a restriction enzyme cleavage site map of pNM14,
Figure 4 is a restriction enzyme cleavage site map of pNM21,
Figure 5 is a restriction enzyme cleavage site map of pNM22, and
Figure 6 illustrates the process for the preparation of a recombinant plasmid containing both the present leader sequence polynucleotide and the β-Galactosidase structural gene, and
Figure 7 is a restriction enzyme cleavage site map of pLEC3.

Materials and methods
Bacterial strains and plasmids
The bacterial strains used were Escherichia coli W5445 (*pro leu thi thr*ᵖ *sup* E44 *lac* Y *ton* A r⁻ m⁻ Strʳ) Pseudomonas putida 2440 (r⁻) and Pseudomonas sp strain RS-16. The plasmids employed were pBR322 (F. Bolivar et al Gene, 1977, 2, 95), pAT153 (A. J. Twigg et al, Nature, 1980, 283, 216) and pKT230 (M. Bagdasarin *et al*, Gene 1981, 16, 237) and pROG5 (R. F. Sherwood *et al*, The Molecular Biology of Yeast, 1979 Cold Spring Harbor Publications).

Media and culture conditions
*E. coli* was routinely cultured in L-broth (1% tryptone, 0.5% yeast extract, 0.5% NaCl). Solidified medium (L-agar) consisted of L-broth with the addition of 2% (w/v) agar (Bacto-Difco). Antibiotic concentrations used for the selection of transformants were 50 µg/ml ampicillin, 15 µg/ml tetracycline and 30 µg/ml kanamycin. In the case of E. coli these were conducted in 2YT liquid medium (1.6% tryptone, 1% yeast extract, 0.5% NaCl) containing 1% glucose, and 0.05% folate where appropriate. The pseudomonads were grown in a minimal salts solution consisting of per litre: $MgSO_4$, 0.05 g; $CaCl_2$, $2H_2O$, 0.05 g; $FeSO_4 \cdot 7H_2O$, 0.005 g; $MnSO_4$, 0.0015 g; $Na_2Mbo_4$, $2H_2O$, 0.0015 g; $KH_2PO_4$; 5 g; $K_2HPO_4{:}3H_2O$, 12 g; glutamate, 10 g. The minimal medium employed for E. coli was M9 medium (J. Miller, Experiments in molecular genetics, Cold Spring Harbor Laboratory, Cold Spring Harbor, New York, 1972).

Purification of DNA
Plasmids were purified from chloramphenicol amplified cultures (D. B. Clewell, J. Bacteriol, 1972, 110, 667) by Brij-lysis (D. B. Clewell et al, Proc. Natl. Acad. Sci., USA, 1969, 62, 1159) and subsequent caesium chloride-ethidium bromide density gradient centrifugation (A. Colman et al, Eur. J. Biochem, 1978, 91, 303). A rapid, small scale plasmid isolation technique (Birnboim et al. Nucl. Acids Res. 1979, 7, 1513) was also employed for screening purposes. Chromosomal DNA from the donor Pseudomonas strain (RS-16) was prepared essentially as described by J. Marmar, J. Mol. Biol, 1961, 3, 208.

Restriction, ligation and transformation methods
Restriction endonucleases and DNA ligase were purchased from Bethesda Research Laboratories and used in the buffers and under the conditions recommended by the supplier. Transformation of *E. coli* was essentially as described by S. N. Cohen et al., Proc. Natl. Acad. Sci., USA, 1972, 69, 2110, while Ps.putida was transformed by the method of M. Bagdasarian and K. N. Timmis, Current Topics in Microbiology and Immunology, Eds. P. H. Hofschneider and W. Goebel, Springer Verlag, Berlin, 1981, p. 47.

7

Agarose gel electrophoresis

Digests were electrophoresed in 0.8% agarose slab gels (10 cm×20 cm×0.5 cm) on a standard vertical system (Raven), employing Tris-borate-EDTA buffer. Electrophoresis of undigested DNA was at 125V, 50 mA for 3 hours, while digested DNA was electrophoresed at 15V, 10 mA for 16 hours. Fragment sizes were estimated by comparison with fragments of λDNA digested with HindIII and λDNA cut with both HindIII and EcoRI. Fragments were isolated from gels using electroelution (M. W. McDonnell et al, Proc. Natl. Acad. Sci, USA, 1977, 74, 4835).

Determination of carboxypeptidase $G_2$ activity

Bacteria were grown in 1 litre batch culture and 100 ml samples taken at various stages in the growth phase. Samples were cooled on ice, centrifuged at 13,000×g for 10 minutes and resuspended and frozen in 5 ml of 0.1 M Tris HCl, pH 7.3 containing 0.2 mM $ZnSO_4$. The cells were disrupted using a MSE Ultrasonic Disintegrator (150 W) at medium frequency, amplitude 2, for three 30-second intervals on ice. Cell debris was removed by centrifugation at 10,000×g for 5 minutes. $CPG_2$ activity was determined after J. L. McCullough et al, J. Biol. Chem, 1971, 246, 7207. A 1 ml reaction cuvette containing 0.9 ml of 0.1 M Tris-HCl, pH 7.3 plus 0.2 mM $ZnSO_4$ and 0.1 ml of 0.6 mM methotrexate was equilibrated at 37°C. Enzyme extract was added to the test cuvette and the decrease in absorbance at 320 nm measured using a Pye-Unicam SP1800 double-beam spectrophotometer. Enzyme activity per ml extract was calculated as Δ 320 nm absorbance/min divided by 8.3, which is equivalent to the hydrolysis of 1 μmol of MTX/min at 37°C. Protein concentration was determined by the method of M. M. Bradford, Anal. Biochem., 1976, 72, 248.

Cell fractionation techniques

Bacterial cultures were grown in the low phosphate medium of H. C. Neu and L. A. Heppel, (J. Biol. Chem., 1964, 240, 3685), supplemented with 100 μg/ml ampicillin, to an $OD_{450}$=1.0. 40 ml of culture was centrifuged at 5000 g for 10 min, washed in 5 ml of 10 mM Tris-HCl pH 7.0, and resuspended in 0.9 ml of 0.58 M sucrose, 0.2 mM DTT, 30 mM Tris-HCl pH 8.0. Conversion to spheroplasts was achieved by the addition of 20 μl of lysozyme (2 mg/ml), 40 μl 0.1 M EDTA, and incubation at 23° for 10 min (H. C. Neu et al, J. Biol. Chem., 1964, 239, 3893). The spheroplasts were placed on ice and 0.1 ml of 30% (w/v) BSA added, followed by 5 ml of sucrose-tris buffer. Sedimentation of the spheroplasts was achieved by centrifugation at 5000 g for 10 min and the supernatant retained as the 'periplasmic' fraction. The pellet was resuspended in 5 ml-10 mM Tris-HCl, 0.2 mM DTT pH 7.0 and sonicated at 20 Kc/sec, 2 Amps for 15 sec. Remaining whole cells were removed by centrifugation at 1000×g for 10 min. Centrifugation at 100000×g for 1 hr, at 4°C, separated the soluble (cytoplasmic) proteins from the particulate (membrane-bound) proteins. The membrane pellet was resuspended in 1 ml of 10 mM Tris-HCl, 0.2 mM DTT, pH 7.0.

$CPG_2$ was assayed as described. Alkaline phosphatase was assayed according to J. Miller, Experiments in Molecular genetics, Cold Spring Harbor Laboratory, Cold Spring Harbor, New York, 1972, NADH oxidase according to M. J. Osborn et al, J. Biol. Chem., 1972, 247, 3962 and glyceraldehyde-3-phosphate dehydrogenase after K. Suzuki et al, FEMS, 1971, 13, 217.

Example 1

Preparation of recombinant plasmid pNM1 (A plasmid containing both the present leader sequence polynucleotide and the $CPG_2$ structural gene).

To isolate the gene for carboxypeptidase $G_2$ together with the leader sequence polynucleotide chromosomal DNA prepared from the Pseudomonas host (strain RS-16) was partially digested with Sau3A and fragments of between 6—8 Md isolated from agarose gels by electroelution. The 'sized' DNA was ligated with alkaline phosphatase treated BamHI cut pBR322, transformed into E. coli W5445, and Ap$^r$ transformants selected. Of the 3,500 Ap$^r$ colonies obtained, approximately 70% were Tc$^s$. Utilisation of a rapid plasmid isolation technique on 50 Ap$^r$ Tc$^s$ transformants demonstrated that 90% of the gene bank harboured plasmids of the expected size. As a further check on the authenticity of the gene bank, the individual clones were screened for the acquisition of a Leu$^+$ phenotype. Two such clones were identified. Both carried a plasmid capable of transforming leuB (B-isopropylmalate dehydrogenase) E. coli mutants to prototrophy.

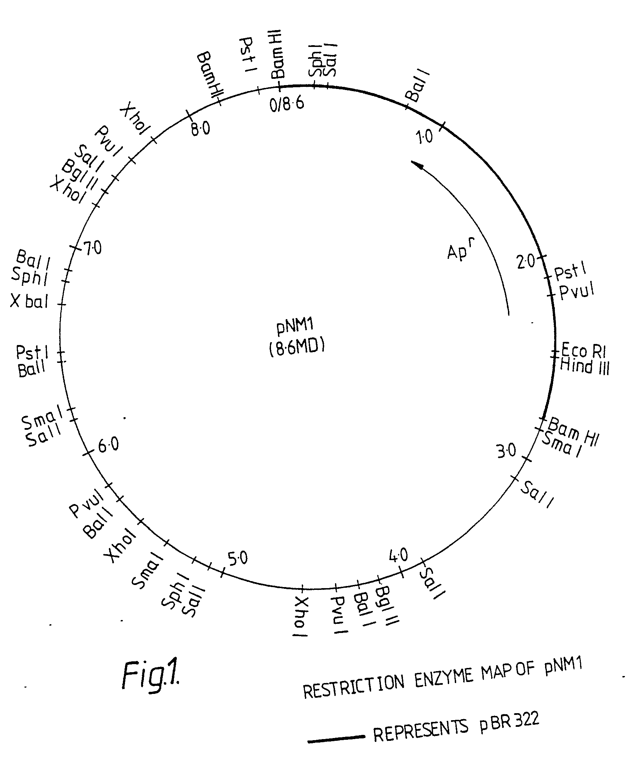

Acquisition of a functional $CPG_2$ gene should enable E. coli to utilise folic acid as a carbon source. The 2,400 gene bank clones were screened for the ability to grow on minimal medium containing folate as the sole source of carbon (i.e. Fol$^+$). A single Fol$^+$ clone was detected and shown to harbour a plasmid capable of transforming plasmid-minus W5445 to the Fol$^+$ phenotype. Classical restriction mapping of this plasmid (pNM1) was undertaken which revealed the presence of a 5.9 Md insert of pseudomonad DNA within pBR322. The restriction enzyme cleavage site map of pNM1 is given in Figure 1. The nucleotide sequence of the leader sequence polynucleotide and the $CPG_2$ structural gene is given in Table 1.

Example 2

Subcloning of plasmid pNM1 to form pNM111

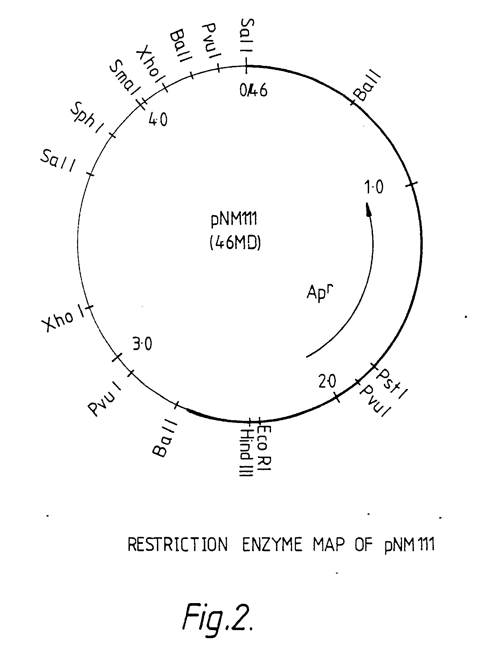

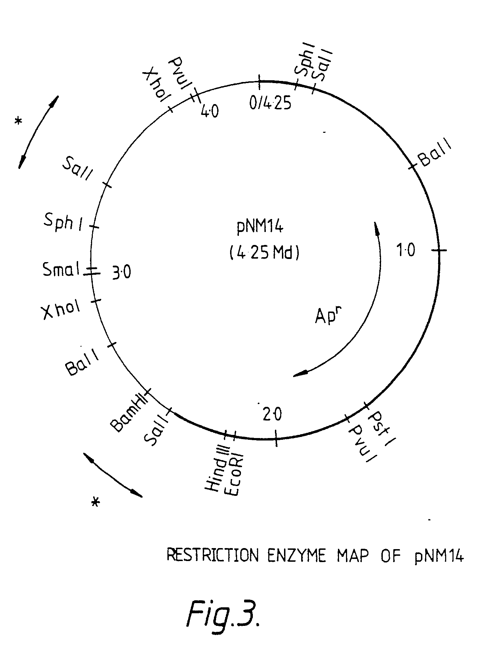

In order to pinpoint the position of the $CPG_2$ gene and the leader sequence polynucleotide within the 5.9 Md insert, subcloning of various restriction enzyme fragments, into pBR322, was undertaken. A functional $CPG_2$ gene was shown not to occur on XhoI or SphI fragments of the pNM1 insert, but was present on a 3.1 Md *Bg*111 fragment. This latter fragment was cloned into the *Bam*H1 site of pBR322 to give pNM11 (6.0 Md). A further reduction in the size of pNM11 was achieved by digesting with *Sa*11 and relegating the resultant fragment to yield pNM111. In addition, plasmids in which the smaller 0.95 Md *Sa*11 fragment had become inserted in the opposite orientation to the parent plasmid (pNM11) were Fol⁻. Taken together these subcloning results indicate that the CPG$_2$ gene and the leader sequence polynucleotide lie between the Bg111 site at 4.14 and the SA11 site at 6.03 on Pnml. Furthermore, the gene contains a *Sph*I (5.17), *Sal*I (5.07) and at least one *Xho*I (4.56 and/or 5.56) site. The restriction enzyme cleavage site map of pNM111 is given in Figure 2.

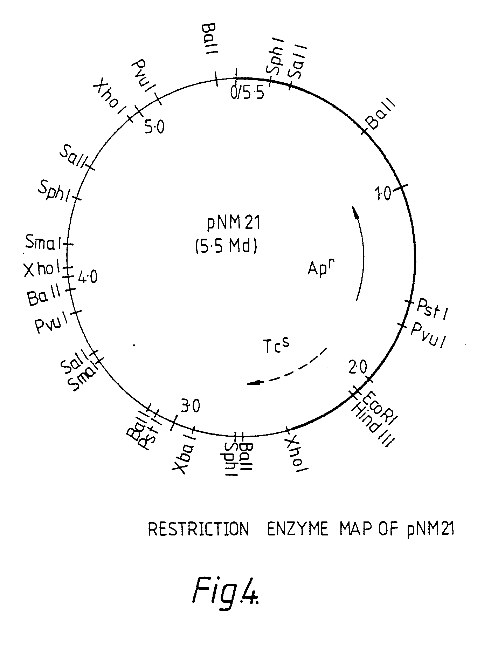

Example 3
Preparation of recombinant plasmid pNM14. (A plasmid containing both the present leader sequence polynucleotide and the CPG$_2$ structural gene)
 The 3.1 Md *Bg*I II fragment from Example 2 above was partially digested with *Sau*3A. These fragments were then cloned into the Bam HI site of pAT153 and transformed into *E. coli* W5445. Of the two Ap$^r$ Tc$^s$ Fol$^+$ colonies obtained, one contained a plasmid which had acquired an extra *Sal* I and *Bam* HI site, this was pNM14. The restriction enzyme cleavage site map of pNM14 is given in Figure 3. Sequencing of the leader sequence polynucleotide and the CPG$_2$ structural gene present in pNM14 gave the nucleotide structure shown in Table 1. DNA sequencing of pNM14 also revealed that the *Sal* I—*Bam* HI fragment was a duplication of a segment of DNA from within the insert (marked * on Figure 3) composed of two contiguous *Sau* 3A fragments.

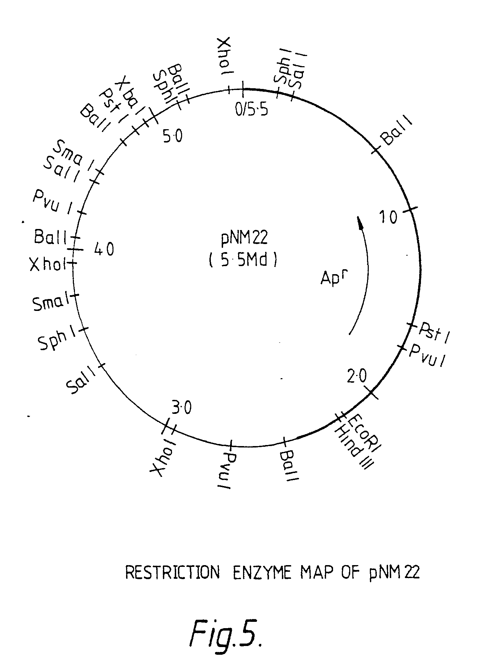

Example 4 and 5
Preparation of recombinant plasmids pNM21 and pNM22 (Plasmids containing both the present leader sequence polynucleotide and the CPG$_2$ structural gene)
 The 3.1 Md *Bg*I II fragment from Example 2 was cloned into the Bam HI site of pAT 153 and transformed into E. coli W5445. Two Ap$^r$ Tc$^s$ Fol$^+$ colonies were obtained, one containing a plasmid pNM21 in which the fragment was inserted in the opposite orientation to pNM1 and one containing a plasmid pNM22 in which the fragment was inserted in the same orientation as pNM1. The restriction enzyme cleavage site maps of pNM21 and pNM22 are given in Figures 4 and 5 respectively.
 The two plasmids, pNM21 and pNM22 both transformed *E. coli* to Fol$^+$, indicating that a pseudomonad promoter was present on the 3.1 Md fragment. However, cells carrying the plasmid pNM21, in which the *Bg*III fragment was cloned in the opposite orientation to pNM1, exhibited more rapid growth with folic acid as the sole carbon source. This difference was clearly visible on agar medium, where colonies developed concentric yellow "halos" of precipitated pteroic acid, the insoluble product of folate hydrolysis.
 Confirmation that pNM21 gave enhanced expression of CPG$_2$ over pNM22, was obtained by assaying enzyme production during batch growth of cells containing either plasmid. (The cells were grown in complex medium supplemented with 1% (w/v) glucose and where appropriate 0.05% (w/v) folic acid. The generation time was 56—66 min. The culture was sampled at hourly intervals and whole cells were disrupted by sonication. Enzyme activity was determined in the centrifugal extract). Results are given in Table 2.
 The expression of CPG$_2$ from the plasmids pNM22 and pNM1 was 2.5 units/litre of culture, representing 0.005% soluble protein. In contrast, expression from pNM21 was 3000—3500 units/litre of culture, which represented 4.7% soluble protein. As the cloned gene is inserted into the *Bam*HI site of pAT153, the observed higher expression of pNM21 is almost certainly due to transcriptional read through from the Tc promoter. The low expression of CPG$_2$ carried on plasmids pNM1 and pNM22 is consistent with the view that Pseudomonas promoters function poorly in *E. coli*. It is also apparent from Table 2 that in the presence of folate there is a two-fold increase in the specific activity of enzyme measured in cell sonicates. This phenomenon has been observed in all experiments, but does not seem to be associated with classical induction of the CPG$_2$ gene, as overall enzyme yield in the presence or absence of folate remains at about 3000 u/litre culture. It in fact reflects a consistent depression in the soluble protein levels measured in sonicates from cells grown in the presence of folate. There is no obvious difference in growth rate of cells grown with folate and the reasons for this result are not clear.

TABLE 2

Carboxypeptidase G production by E. coli W5445, containing the plasmids pNM1, pNM21 and pNM22

| Culture | Carboxypeptidase $G_2$ specific activity (U/MG soluble protein) | | | | | |
|---|---|---|---|---|---|---|
| | pNM1 | | pNM22 | | pNM21 | |
| Age (hr) | −Fol | +Fol | −Fol | +Fol | −Fol | +Fol |
| 1 | — | — | — | — | 11.5 | 13.4 |
| 2 | — | — | — | — | 12.9 | 9.6 |
| 3 | .008 | .005 | .010 | .019 | 13.9 | 23.3 |
| 4 | .009 | .011 | .015 | .013 | 12.3 | 26.9 |
| 5 | .007 | .019 | .016 | .016 | 11.5 | 25.6 |
| 6 | .005 | .024 | .014 | .023 | 13.7 | 24.1 |
| 7 | .015 | .029 | .024 | .043 | 13.2 | 20.6 |
| 8 | .013 | .028 | .024 | .046 | 13.0 | 23.6 |

Expression of the cloned gene in *Ps. putida*

The observation that the $CPG_2$ gene was expressed in *E. coli* regardless of the orientation of the gene within the vector suggested that the promoter region of the $CPG_2$ gene had been cloned with the structural gene and the leader sequence polynucleotide. The low expression of $CPG_2$ within *E. coli* from its natural promoter (pNM1, pNM22, pNM111) confirmed other findings that *Pseudomonas* promoters are poorly recognised by *E. coli* RNA polymerases. It would be expected that if the gene was introduced back into a pseudomonad cellular environment, then improved expression from the *Pseudomonas* promoter should result. The 3.1 Md *Bg*III fragment was subcloned into the *Pseudomonas* cloning vector pKT230 at its single *Bam*HI site. Two plasmids were obtained, pNM31 and pNM32 representing the two possible orientations of the cloned gene. These plasmids were transformed into *Ps. putida* 2440 by the method of Bagdasarian and Timmis. Pseudomonas cells carrying both plasmids were cultured in minimal salts medium and enzyme production monitored.

Yields of 500—1000 units/litre of culture were obtained regardless of gene orientation within the plasmid. Specific activity of the enzyme in cell sonicates was 1.5 to 4.0 U/mg protein representing 0.3 to 0.7% soluble protein (compared with <0.05% soluble protein in the donor strain RS-16). This result strongly indicates that the $CPG_2$ promoter is present and operated in a pseudomonad background. When the same plasmids were transformed into *E. coli* W5445 12—40 Units/litre were found at specific activity <0.07 U/mg (<0.01% soluble protein).

Periplasmic localisation of $CPG_2$

There is evidence that $CPG_2$ is located in or near the periplasmic space of *Pseudomonas* strain RS-16. Pteroic acid, the product of $CPG_2$ hydrolysis of folic acid is extremely insoluble and is found predminantly outside the cell in both liquid and solid media. Exogeneous pteroic acid is also seen in *E. coli* cultures containing the cloned gene when folic acid is present in the medium. This is clearly demonstrated by the "halo" of precipitated pteroic acid observed around colonies carrying plasmids in which expression of $CPG_2$ is from the Tc promoter of pBR322 (e.g. pNM21).

The localisation of $CPG_2$ produced by *E. coli* cells carrying pNM21 was examined by the separation of cellular proteins into cytoplasmic, periplasmic, and whole membrane fractions. As a control, levels of three marker enzymes, alkaline phosphatase (periplasmic), glyceraldehyde-3-phosphate dehydrogenase (cytoplasmic) and $NADH \cdot O_2$ oxidoreductase (membrane-bound), were also determined. As can be seen from Table 3 97% of the $CPG_2$ activity occurs in the periplasm, equivalent to the marker periplasmic enzyme, alkaline phosphatase. This confirms the presence in pNM21 of a leader sequence polynucleotide next to the $CPG_2$ gene that codes for a signal polypeptide according to this invention that promotes the secretion of $CPG_2$ from the cytoplasm into the periplasmic space.

Carboxypeptidase $G_2$ synthesised in *E. coli*

The specific activity of $CPG_2$ in crude cell extracts of cells carrying pNM21 was 50-fold higher than equivalent extracts from *Pseudomonas* strain RS-16. To determine whether the cloned gene=product in *E.*

*coli* had the same properties as $CPG_2$ from the pseudomonad, enzyme was purified from *E. coli* carrying pNM21. The specific activity of purified $CPG_2$ (single band SDS-PAGE) was 535 U/mg of protein, which compares to 550 U/mg of protein from the pseudomonad. $CPG_2$ purified from *E. coli* clone pNM21 co-chromatographed with $CPG_2$ from *Pseudomonas* strain RS-16 at a sub-unit molecular weight value of 42,000 daltons. Km values using methotrexate as substrate were $7.4 \times 10^{-6}M$ and $8.0 \times 10^{-6}M$ respectively. In addition, antiserum raised against the *Pseudomonas* enzyme indicated immunological identity between the *E. coli* and *Pseudomonas* $CPG_2$, as a confluent precipitation line was formed on Ouchterlony double diffusion analysis.

TABLE 3
Localisation of carboxypeptidase

| Fraction | CPG$_2$ | Enzyme activity | | |
| --- | --- | --- | --- | --- |
| | | AP | GAPDH | NADHOX |
| Periplasmic | 97.0 | 97.1 | 6.8 | 0.25 |
| Cytoplasmic | 2.6 | 2.3 | 93 | 8.4 |
| Membrane-bound | 0.4 | 0.6 | 0.2 | 89.1 |

AP=Alkaline phosphatase
GAPDH=Glyceraldehyde-3-phosphate dehydrogenase
NADHOX=NADH · $O_2$ oxidoreductase

Example 6
Preparation of a recombinant plasmid containing both the present leader sequence polynucleotide and the β-Galactosidase structural gene

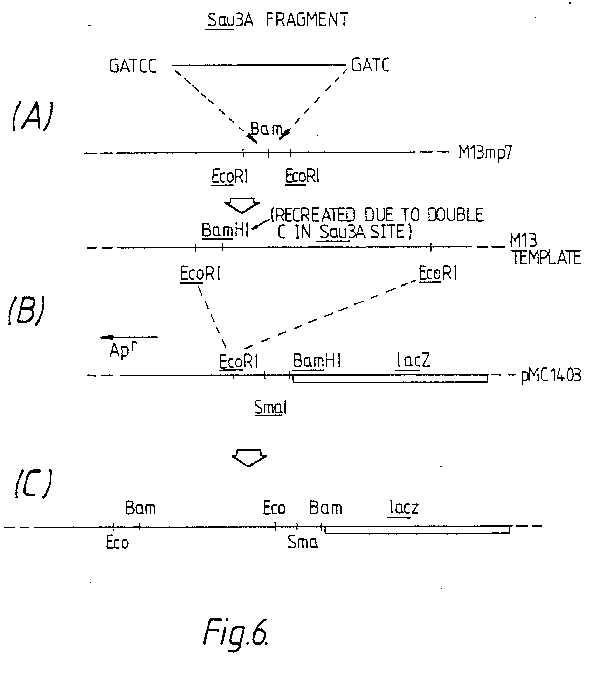

Plasmid pNM14 (Example 3) was treated with *Sau* 3A (GATC) and the fragments were cloned into the *Bam* HI site of M13 mp7 template DNA (single stranded DNA (Step A of Figure 6). The product carrying a 318bp *Sau* 3A fragment coding for the present signal polypeptide and the first 22 amino acids of $CPG_2$ (nucleotide sequence of this fragment shown in Table 4) was selected and made double stranded. The DNA coding for the signal polypeptide (and the first part of $CPG_2$) was then excised as an *Eco*RI fragment. This *Eco*RI fragment was then cloned into the promoter cloning vector E. coli pMC1403 (M. J. Casadaban *et al*, J. Bacteriol, 1980, 143, 971), which carries only the structural gene (lac Z) for β-galactosidase (i.e. no promoter and no ATG start codon) (Steps B and C of Figure 6). Plasmids were obtained in which the *Eco*RI fragment had inserted in both orientations, however, only those in which fusion of the $CPG_2$ sequence to the β-galactosidase sequence had occurred (i) yielded a 0.34 Kb fragment upon digestion with *Bam*HI; (ii) enabled the host cell to hydrolyse the colourless lactose analogue, BCIG, and impart a blue colouration to colonies. The 0.34 Kb *Bam*HI fragment has been recloned into M13mp7 and sequenced to confirm that fusion has occurred. The 'precursor' fusion produced will consist of the signal peptide, the first 22 amino acids of $CPG_2$, 6 amino acids derived from the M13mp7 and pMC1403 linker units, and·β-galactosidase from its 8th amino acid onward.

Localisation experiments have been performed on cells carrying a plasmid coding for the 'fusion gene' where the cellular proteins have been fractionated into periplasmic, cytoplasmic and membrane fractions. In these experiments an organism (E. coli MC 1061) which is deleted for the lac Z gene was grown in phosphate medium (H. C. Neu et al, J. Biol. Chem., 1964, 240, 3685) and periplasmic enzymes were released from the harvested cells by conversion to spheroplasts. Separation of soluble proteins (cytoplasmic) from particulate proteins (membrane band) was achieved by sonicating the harvested spheroplasts and subsequent centrifugation at 100,000 g for 1 hr, to sediment the cell membrane (T. J. Silhary et al, Proc. Natl. Acad. Sci. USA, 1976, 73, 3423).

The results given in Table 5 demonstrate the presence of 50% of the β-galactosidase activity in the periplasmic space. This result is in direct contrast to similar work involving fusion of other periplasmic protein signal sequences to β-galactosidase, where the fusion proteins are not exported, but become jammed in the membrane (P. J. Bassford et al, J. Bacteriol, 1979, 139, 19 and S. D. Emr et al, J. Cell, Biol., 1980, 86, 701).

**TABLE 4**

The polynucleotide sequence of the 318 bp *Sau* 3A fragment from
recombinant plasmid pNM14

| | | | | | | | | |
|---|---|---|---|---|---|---|---|---|
| 5'—G | ATC | CAC | GCA | CTG | AAG | GCG | CGC | GGC |
| AAG | ACG | CGC | GGC | GTG | GCG | ACG | CTG | TGC |
| ATC | GGC | GGG | GGC | GAA | GGC | ACC | GCA | GTG |
| GCA | CTC | GAT | TGC | TAT | AAG | AAC | CAT | GGC |
| TGG | GGA | CGC | CCG | ACA | ACA | GGC | GTC | CAC |
| CAG | CTT | TTT | TCA | TTC | CGA | CAA | CCC | GAA |
| CGA | ACA | ATG | CGT | AGA | GCA | GGA | GAT | TCC |

| | | | | | | | | |
|---|---|---|---|---|---|---|---|---|
| | Met | Arg | Pro | Ser | Ile | His | Arg | Thr |
| | ATG | CGC | CCA | TCC | ATC | CAC | CGC | ACA |
| Ala | Ile | Ala | Ala | Val | Leu | Ala | Thr | Ala |
| GCC | ATC | GCC | GCC | GTG | CTG | GCC | ACC | GCC |
| Phe | Val | Ala | Gly | Thr | Ala | Leu | Ala | Gln |
| TTC | GTG | GCG | GGC | ACC | GCC | CTG | GCC | CAG |
| Lys | Arg | Asp | Asn | Val | Leu | Phe | Gln | Ala |
| AAG | CGC | GAC | AAC | GTG | ·CTG | TTC | CAG | GCA |
| Ala | Thr | Asp | Glu · | Gln | Pro | Ala | Val | Ile |
| GCT | ACC | GAC | GAG | CAG | CCG | GCC | GTG | ATC |

NB. This fragment carries the leader sequence coding for the signal polypeptide, a part of the $CPG_2$ structural gene coding for the first 22 amino acids of the protein, the ATG start codon, the $CPG_2$ ribosome binding site (AGGA) and other components of the $CPG_2$ promoter region.

**TABLE 5**

Localisation of signal peptide—β-galactosidase fusion protein

| | | % Localisation[a] | | |
|---|---|---|---|---|
| | CPG2/ß-gal | AP | GAPDH | NADHOX |
| Periplasmic | 50.3 | 97.3 | 3.4 | 0.4 |
| Cytoplasmic | 30.9 | 2.5 | 95.3 | 8.2 |
| Membrane-bound | 18.8 | 0.2 | 1.3 | 89.4 |

[a]=average results from 4 experiments
CPG2/ß-gal=Carboxypeptidase G2-β-galactosidase fusion protein
AP=Alkaline phosphatase
GAPDH=Glyceraldehyde-3-phosphate dehydrogenase
NADHOX=NADH · $O_2$ Oxidoreductase

Example 7
Preparation of a recombinant plasmid, containing both the present leader sequence polynucleotide and the $CPG_2$ structural gene, able to replicate in *E. coli* and *S. serevisiae*

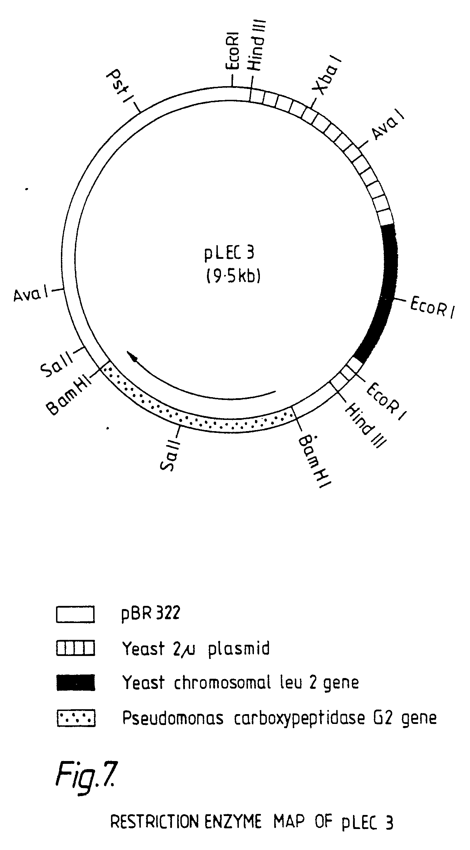

A 2.03 kilobase *Bam*HI fragment coding for the present signal polypeptide and the entire $CPG_2$ molecule was cloned in both orientations into the *Bam*HI site of an *E. coli/S. cerevisiae* shuttle vector pROG5 (R. F. Sherwood and R. K. Gibson, The Molecular Biology of Yeast, 1979, Cold Spring Harbor Publications) to give plasmids pLEC3 and pLEC4 (Figure 7). These plasmids were transformed into *S. cerevisiae* strain LL20 by the lithium acetate induced transformation method described by Ito *et al.*, J. Bact., 1983, *153*, 163. Yields equivalent to 10—20 units/litre of culture volume were obtained regardless of gene

**0 121 352**

orientation within the plasmid. Specific activity of the enzyme in total cell extracts was 0.2—0.3 u/mg protein representing 0.005% soluble protein. This level of expression from the pseudomonad promoter in a yeast background is similar to the level found when the gene was reading from its own promoter in *E. coli* (0.01% soluble protein).

Localisation experiments have been performed on yeast cells carrying the above plasmids by sphaeroplasting the cells using standard techniques described by J. B. D. Beggs, Nature, 1978, *275*, 105. Periplasmic enzymes, localised outside of the cell membrane, were released when the cell wall was removed. The osmotic stabiliser (1.2M sorbitol) was then replaced by 0.1M Tris-HCl buffer, pH 7.3 containing 0.2 mM $ZnCl_2$ to lyse the sphaeroplasts and the whole centrifuged at $100,000 \times g$ for 1 hour to separate proteins in the soluble cytoplasmic fraction from membrane bound proteins. The results in Table 6 demonstrate the presence of 64% of the $CPG_2$ activity in the periplasmic fraction and a further 16% associated with the cell membrane fraction.

TABLE 6

Localisation of $CPG_2$ in *S. cerevisiae*

|  | % $CPG_2$ activity |
| --- | --- |
| Periplasmic | 64 |
| Cytoplasmic | 20 |
| Membrane bound | 16 |

**Claims**

1. A recombinant DNA transfer vector comprising a leader sequence polynucleotide downstream of and in reading phase with a bacterial or yeast promoter and a prokaryotic ribosome binding site and upstream of and in reading phase with a structural gene, characterised in that the leader sequence polynucleotide codes for a signal polypeptide of formula I

Met-Arg-Pro-Ser-Ile-His-Arg-Thr-
Ala-Ile-Ala-Ala-Val-Leu-Ala-Thr-          I
Ala-Phe-Val-Ala-Gly-Thr

2. A recombinant DNA transfer vector according to claim 1 characterised in that the leader sequence polynucleotide is of formula II

| 5'— | ATG | CGC | CCA | TCC | ATC | CAC | CGC | ACA | |
| --- | --- | --- | --- | --- | --- | --- | --- | --- | --- |
|  | GCC | ATC | GCC | GCC | GTG | CTG | GCC | ACC | II |
|  | GCC | TTC | GTG | GCG | GGC | ACC | —3'. | | |

3. A recombinant DNA transfer vector according to either claim 1 or claim 2 characterised in that the structural gene codes for Pseudomonas carboxypeptidase $G_2$ ($CPG_2$).

4. A recombinant DNA transfer vector according to either claim 1 or claim 2 characterised in that the structural gene codes for a protein or polypeptide other than Pseudomonas carboxypeptidase $G_2$.

5. A recombinant DNA transfer vector according to claim 4 characterised in that the structural gene codes for a prokaryotic protein other than Pseudomonas carboxypeptidase $G_2$.

6. A recombinant DNA transfer vector according to claim 5 characterised in that the structural gene codes for E. Coli β-galactosidase.

7. A recombinant DNA transfer vector according to claim 3 comprising a polynucleotide of formula

|  | 1 | | | | | | | |
| --- | --- | --- | --- | --- | --- | --- | --- | --- |
|  | Met | Arg | Pro | Ser | Ile | His | Arg | Thr |
| 5'— | ATG | CGC | CCA | TCC | ATC | CAC | CGC | ACA |
|  | 10 | | | | | | | |
| Ala | Ile | Ala | Ala | Val | Leu | Ala | Thr | Ala |
| GCC | ATC | GCC | GCC | GTG | CTG | GCC | ACC | GCC |
|  | 20 | | | | | | | |
| Phe | Val | Ala | Gly | Thr | Ala | Leu | Ala | Gln |
| TTC | GTG | GCG | GGC | ACC | GCC | CTG | GCC | CAG |

13

# 0 121 352

|       |       |       | 30<br>Asn | Val   | Leu   | Phe   | Gln   | Ala   |
|-------|-------|-------|-------|-------|-------|-------|-------|-------|
| Lys   | Arg   | Asp   | Asn   | Val   | Leu   | Phe   | Gln   | Ala   |
| AAG   | CGC   | GAC   | AAC   | GTG   | CTG   | TTC   | CAG   | GCA   |
| Ala   | Thr   | Asp   | Glu   | 40<br>Gln | Pro | Ala | Val | Ile |
| GCT   | ACC   | GAC   | GAG   | CAG   | CCG   | GCC   | GTG   | ATC   |
| Lys   | Thr   | Leu   | Glu   | Lys   | 50<br>Leu | Val | Asn | Ile |
| AAG   | ACG   | CTG   | GAG   | AAG   | CTG   | GTC   | AAC   | ATC   |
| Glu   | Thr   | Gly   | Thr   | Gly   | Asp   | 60<br>Ala | Glu | Gly |
| GAG   | ACC   | GGC   | ACC   | GGT   | GAC   | GCC   | GAG   | GGC   |
| Ile   | Ala   | Ala   | Ala   | Gly   | Asn   | Phe   | 70<br>Leu | Glu |
| ATC   | GCC   | GCT   | GCG   | GGC   | AAC   | TTC   | CTC   | GAG   |
| Ala   | Glu   | Leu   | Lys   | Asn   | Leu   | Gly   | Phe   | 80<br>Thr |
| GCC   | GAG   | CTC   | AAG   | AAC   | CTC   | GGC   | TTC   | ACG   |
| Val   | Thr   | Arg   | Ser   | Lys   | Ser   | Ala   | Gly   | Leu   |
| GTC   | ACG   | CGA   | AGC   | AAG   | TCG   | GCC   | GGC   | CTG   |
| 90<br>Val | Val | Gly | Asp | Asn | Ile | Val | Gly | Lys |
| GTG   | GTG   | GGC   | GAC   | AAC   | ATC   | GTG   | GGC   | AAG   |
| Ile   | 100<br>Lys | Gly | Arg | Gly | Gly | Lys | Asn | Leu |
| ATC   | AAG   | GGC   | CGC   | GGC   | GGC   | AAG   | AAC   | CTG   |
| Leu   | Leu   | 110<br>Met | Ser | His | Met | Asp | Thr | Val |
| CTG   | CTG   | ATG   | TCG   | CAC   | ATG   | GAC   | ACC   | GTC   |
| Tyr   | Leu   | Lys   | 120<br>Gly | Ile | Leu | Ala | Lys | Ala |
| TAC   | CTC   | AAG   | GGC   | ATT   | CTC   | GCG   | AAG   | GCC   |
| Pro   | Phe   | Arg   | Val   | 130<br>Glu | Gly | Asp | Lys | Ala |
| CCG   | TTC   | CGC   | GTC   | GAA   | GGC   | GAC   | AAG   | GCC   |
| Tyr   | Gly   | Pro   | Gly   | Ile   | 140<br>Ala | Asp | Asp | Lys |
| TAC   | GGC   | CCG   | GGC   | ATC   | GCC   | GAC   | GAC   | AAG   |
| Gly   | Gly   | Asn   | Ala   | Val   | Ile   | 150<br>Leu | His | Thr |
| GGC   | GGC   | AAC   | GCG   | GTC   | ATC   | CTG   | CAC   | ACG   |
| Leu   | Lys   | Leu   | Leu   | Lys   | Glu   | Tyr   | 160<br>Gly | Val |
| CTC   | AAG   | CTG   | CTG   | AAG   | GAA   | TAC   | GGC   | GTG   |
| Arg   | Asp   | Tyr   | Gly   | Thr   | Ile   | Thr   | Val   | 170<br>Leu |
| CGC   | GAC   | TAC   | GGC   | ACC   | ATC   | ACC   | GTG   | CTG   |
| Phe   | Asn   | Thr   | Asp   | Glu   | Glu   | Lys   | Gly   | Ser   |
| TTC   | AAC   | ACC   | GAC   | GAG   | GAA   | AAG   | GGT   | TCC   |

14

| 180 | | | | | | | | |
|---|---|---|---|---|---|---|---|---|
| Phe | Gly | Ser | Arg | Asp | Leu | Ile | Gln | Glu |
| TTC | GGC | TCG | CGC | GAC | CTG | ATC | CAG | GAA |

| | 190 | | | | | | | |
|---|---|---|---|---|---|---|---|---|
| Glu | Ala | Lys | Leu | Ala | Asp | Tyr | Val | Leu |
| GAA | GCC | AAG | CTG | GCC | GAC | TAC | GTG | CTC |

| | | 200 | | | | | | |
|---|---|---|---|---|---|---|---|---|
| Ser | Phe | Glu | Pro | Thr | Ser | Ala | Gly | Asp |
| TCC | TTC | GAG | CCC | ACC | AGC | GCA | GGC | GAC |

| | | | 210 | | | | | |
|---|---|---|---|---|---|---|---|---|
| Glu | Lys | Leu | Ser | Leu | Gly | Thr | Ser | Gly |
| GAA | AAA | CTC | TCG | CTG | GGC | ACC | TCG | GGC |

| | | | | 220 | | | | |
|---|---|---|---|---|---|---|---|---|
| Ile | Ala | Tyr | Val | Gln | Val | Asn | Ile | Thr |
| ATC | GCC | TAC | GTG | CAG | GTC | AAC | ATC | ACC |

| | | | | | 230 | | | |
|---|---|---|---|---|---|---|---|---|
| Gly | Lys | Ala | Ser | His | Ala | Gly | Ala | Ala |
| GGC | AAG | GCC | TCG | CAT | GCC | GGC | GCC | GCG |

| | | | | | | 240 | | |
|---|---|---|---|---|---|---|---|---|
| Pro | Glu | Leu | Gly | Val | Asn | Ala | Leu | Val |
| CCC | GAG | CTG | GGC | GTG | AAC | GCG | CTG | GTC |

| | | | | | | | 250 | |
|---|---|---|---|---|---|---|---|---|
| Glu | Ala | Ser | Asp | Leu | Val | Leu | Arg | Thr |
| GAG | GCT | TCC | GAC | CTC | GTG | CTG | CGC | ACG |

| | | | | | | | | 260 |
|---|---|---|---|---|---|---|---|---|
| Met | Asn | Ile | Asp | Asp | Lys | Ala | Lys | Asn |
| ATG | AAC | ATC | GAC | GAC | AAG | GCG | AAG | AAC |

| | | | | | | | | |
|---|---|---|---|---|---|---|---|---|
| Leu | Arg | Phe | Asn | Trp | Thr | Ile | Ala | Lys |
| CTG | CGC | TTC | AAC | TGG | ACC | ATC | GCC | AAG |

| 270 | | | | | | | | |
|---|---|---|---|---|---|---|---|---|
| Ala | Gly | Asn | Val | Ser | Asn | Ile | Ile | Pro |
| GCC | GGC | AAC | GTC | TCG | AAC | ATC | ATC | CCC |

| | 280 | | | | | | | |
|---|---|---|---|---|---|---|---|---|
| Ala | Ser | Ala | Thr | Leu | Asn | Ala | Asp | Val |
| GCC | AGC | GCC | ACG | CTG | AAC | GCC | GAC | GTG |

| | | 290 | | | | | | |
|---|---|---|---|---|---|---|---|---|
| Arg | Tyr | Ala | Arg | Asn | Glu | Asp | Phe | Asp |
| CGC | TAC | GCG | CGC | AAC | GAG | GAC | TTC | GAC |

| | | | 300 | | | | | |
|---|---|---|---|---|---|---|---|---|
| Ala | Ala | Met | Lys | Thr | Leu | Glu | Glu | Arg |
| GCC | GCC | ATG | AAG | ACG | CTG | GAA | GAG | CGC |

| | | | | 310 | | | | |
|---|---|---|---|---|---|---|---|---|
| Ala | Gln | Gln | Lys | Lys | Leu | Pro | Glu | Ala |
| GCG | CAG | CAG | AAG | AAG | CTG | CCC | GAG | GCC |

| | | | | | 320 | | | |
|---|---|---|---|---|---|---|---|---|
| Asp | Val | Lys | Val | Ile | Val | Thr | Arg | Gly |
| GAC | GTG | AAG | GTG | ATC | GTC | ACG | CGC | GGC |

| | | | | | | 330 | | |
|---|---|---|---|---|---|---|---|---|
| Arg | Pro | Ala | Phe | Asn | Ala | Gly | Glu | Gly |
| CGC | CCG | GCC | TTC | AAT | GCC | GGC | GAA | GGC |

| | | | | | | | 340 | |
|---|---|---|---|---|---|---|---|---|
| Gly | Lys | Lys | Leu | Val | Asp | Lys | Ala | Val |
| GGC | AAG | AAG | CTG | GTC | GAC | AAG | GCG | GTG |

| | | | | | | | | 350 |
|---|---|---|---|---|---|---|---|---|
| Ala | Tyr | Tyr | Lys | Glu | Ala | Gly | Gly | Thr |
| GCC | TAC | TAC | AAG | GAA | GCC | GGC | GGC | ACG |

| Leu | Gly | Val | Glu | Glu | Arg | Thr | Gly | Gly |
|---|---|---|---|---|---|---|---|---|
| CTG | GGC | GTG | GAA | GAG | CGC | ACC | GGC | GGC |

| 360 | | | | | | | | |
|---|---|---|---|---|---|---|---|---|
| Gly | Thr | Asp | Ala | Ala | Tyr | Ala | Ala | Leu |
| GGC | ACC | GAC | GCG | GCC | TAC | GCC | GCG | CTC |

| | 370 | | | | | | | |
|---|---|---|---|---|---|---|---|---|
| Ser | Gly | Lys | Pro | Val | Ile | Glu | Ser | Leu |
| TCA | GGC | AAG | CCA | GTG | ATC | GAG | AGC | CTG |

| | | 380 | | | | | | |
|---|---|---|---|---|---|---|---|---|
| Gly | Leu | Pro | Gly | Phe | Gly | Tyr | His | Ser |
| GGC | CTG | CCG | GGC | TTC | GGC | TAC | CAC | AGC |

| | | | 390 | | | | | |
|---|---|---|---|---|---|---|---|---|
| Asp | Lys | Ala | Glu | Tyr | Val | Asp | Ile | Ser |
| GAC | AAG | GCC | GAG | TAC | GTG | GAC | ATC | AGC |

| | | | | 400 | | | | |
|---|---|---|---|---|---|---|---|---|
| Ala | Ile | Pro | Arg | Arg | Leu | Tyr | Met | Ala |
| GCG | ATT | CCG | CGC | CGC | CTG | TAC | ATG | GCT |

| | | | | | 410 | | | |
|---|---|---|---|---|---|---|---|---|
| Ala | Arg | Leu | Ile | Met | Asp | Leu | Gly | Ala |
| CGC | CGC | CTG | ATC | ATG | GAT | CTG | GGC | GCC |

| Gly | Lys | | |
|---|---|---|---|
| GGC | AAG | TGA | —3' |

8. A recombinant DNA transfer vector according to any preceding claim characterised in that the transfer vector is a plasmid.

9. A microorganism transformed by a transfer vector characterised in that the transfer vector is a recombinant DNA transfer vector according to any one of the preceding claims 1 to 8.

10. A microorganism according to claim 9 which is a bacterium of the species E. coli or Pseudomonas or a yeast of the species Saccharomyces cerevisiae.

11. A process for the preparation of a gene product characterised by

(a) culturing a microorganism according to either claim 9 or claim 10 in a culture medium to produce the gene product in the culture medium or the periplasmic space of the microorganism, and

(b) isolating the gene product from the culture medium or the periplasmic space of the microorganism.

12. A process according to claim 11 characterised in that the gene product is Pseudomonas carboxypeptidase $G_2$.

13. A process according to claim 11 characterised in that the gene product is a protein or polypeptide other than Pseudomonas carboxypeptidase $G_2$.

14. A process according to claim 13 characterised in that the gene product is a prokaryotic protein other than Pseudomonas carboxypeptidase $G_2$.

15. A process according to claim 14 characterised in that the gene product is E. coli β-galactosidase.

**Patentansprüche**

1. Rekombinanter DNA-Transfervektor, enthaltend ein Leitsequenz-polynukleotid stromabwärts von und in Lesephase mit einem Bakterien- oder Hefepromotor und einer prokaryotischen Ribosomen-Bindestelle und stromaufwärts von und in Lesephase mit einem Strukturgen, dadurch gekennzeichnet, daß das Leitsequenz-polynukleotid für ein Signalpolypeptid der Formel I codiert

Met-Arg-Pro-Ser-Ile-His-Arg-Thr-
Ala-Ile-Ala-Ala-Val-Leu-Ala-Thr-          I
Ala-Phe-Val-Ala-Gly-Thr.

2. Rekombinanter DNA-Transfervektor nach Anspruch 1, dadurch gekennzeichnet, daß das Leitsequenz-polynukleotid die Formel II aufweist

| 5'— | ATG | CGC | CCA | TCC | ATC | CAC | CGC | ACA | |
|-----|-----|-----|-----|-----|-----|-----|-----|-----|-----|
| | GCC | ATC | GCC | GCC | GTG | CTG | GCC | ACC | II |
| | GCC | TTC | GTG | GCG | GGC | ACC | —3'. | | |

3. Rekombinanter DNA-Transfervektor nach Anspruch 1 oder 2, dadurch gekennzeichnet, daß das Strukturgen für Pseudomonas-Carboxypeptidase $G_2$ (CPG$_2$) codiert.

4. Rekombinanter DNA-Transfervektor nach Anspruch 1 oder 2, dadurch gekennzeichnet, daß das Sturkturgen für ein anderes Protein oder Polypeptid codiert als Pseudomonas-Carboxypeptidase $G_2$.

5. Rekombinanter DNA-Transfervektor nach Anspruch 4, dadurch gekennzeichnet, daß das Strukturgen für ein anderes prokaryotisches Protein als Pseudomonas-Carboxypeptidase $G_2$ codiert.

6. Rekombinanter DNA-Transfervektor nach Anspruch 5, dadurch gekennzeichnet, daß das Strukturgen für β-Galactosidase aus E. coli codiert.

7. Rekombinanter DNA-Transfervektor nach Anspruch 3, enthaltend ein Polynukleotid der Formel

| | 1 | | | | | | | |
|---|---|---|---|---|---|---|---|---|
| | Met | Arg | Pro | Ser | Ile | His | Arg | Thr |
| 5'— | ATG | CGC | CCA | TCC | ATC | CAC | CGC | ACA |
| | 10 | | | | | | | |
| Ala | Ile | Ala | Ala | Val | Leu | Ala | Thr | Ala |
| GCC | ÁTC | GCC | GCC | GTG | CTG | GCC | ACC | GCC |
| | | 20 | | | | | | |
| Phe | Val | Ala | Gly | Thr | Ala | Leu | Ala | Gln |
| TTC | GTG | GCG | GGC | ACC | GCC | CTG | GCC | CAG |
| | | | 30 | | | | | |
| Lys | Arg | Asp | Asn | Val | Leu | Phe | Gln | Ala |
| AAG | CGC | GAC | AAC | GTG | CTG | TTC | CAG | GCA |
| | | | | 40 | | | | |
| Ala | Thr | Asp | Glu | Gln | Pro | Ala | Val | Ile |
| GCT | ACC | GAC | GAG | CAG | CCG | GCC | GTG | ATC |
| | | | | | 50 | | | |
| Lys | Thr | Leu | Glu | Lys | Leu | Val | Asn | Ile |
| AAG | ACG | CTG | GAG | AAG | CTG | GTC | AAC | ATC |
| | | | | | | 60 | | |
| Glu | Thr | Gly | Thr | Gly | Asp | Ala | Glu | Gly |
| GAG | ACC | GGC | ACC | GGT | GAC | GCC | GAG | GGC |
| | | | | | | | 70 | |
| Ile | Ala | Ala | Ala | Gly | Asn | Phe | Leu | Glu |
| ATC | GCC | GCT | GCG | GGC | AAC | TTC | CTC | GAG |
| | | | | | | | | 80 |
| Ala | Glu | Leu | Lys | Asn | Leu | Gly | Phe | Thr |
| GCC | GAG | CTC | AAG | AAC | CTC | GGC | TTC | ACG |
| Val | Thr | Arg | Ser | Lys | Ser | Ala | Gly | Leu |
| GTC | ACG | CGA | AGC | AAG | TCG | GCC | GGC | CTG |

17

|  |  |  |  |  |  |  |  |  |
|---|---|---|---|---|---|---|---|---|
| 90 |  |  |  |  |  |  |  |  |
| Val | Val | Gly | Asp | Asn- | Ile | Val | Gly | Lys |
| GTG | GTG | GGC | GAC | AAC | ATC | GTG | GGC | AAG |
|  | 100 |  |  |  |  |  |  |  |
| Ile | Lys | Gly | Arg | Gly | Gly | Lys | Asn | Leu |
| ATC | AAG | GGC | CGC | GGC | GGC | AAG | AAC | CTG |
|  |  | 110 |  |  |  |  |  |  |
| Leu | Leu | Met | Ser | His | Met | Asp | Thr | Val |
| CTG | CTG | ATG | TCG | CAC | ATG | GAC | ACC | GTC |
|  |  |  | 120 |  |  |  |  |  |
| Tyr | Leu | Lys | Gly | Ile | Leu | Ala | Lys | Ala |
| TAC | CTC | AAG | GGC | ATT | CTC | GCG | AAG | GCC |
|  |  |  |  | 130 |  |  |  |  |
| Pro | Phe | Arg | Val | Glu | Gly | Asp | Lys | Ala |
| CCG | TTC | CGC | GTC | GAA | GGC | GAC | AAG | GCC |
|  |  |  |  |  | 140 |  |  |  |
| Tyr | Gly | Pro | Gly | Ile | Ala | Asp | Asp | Lys |
| TAC | GGC | CCG | GGC | ATC | GCC | GAC | GAC | AAG |
|  |  |  |  |  |  | 150 |  |  |
| Gly | Gly | Asn | Ala | Val | Ile | Leu | His | Thr |
| GGC | GGC | AAC | GCG | GTC | ATC | CTG | CAC | ACG |
|  |  |  |  |  |  |  | 160 |  |
| Leu | Lys | Leu | Leu | Lys | Glu | Tyr | Gly | Val |
| CTC | AAG | CTG | CTG | AAG | GAA | TAC | GGC | GTG |
|  |  |  |  |  |  |  |  | 170 |
| Arg | Asp | Tyr | Gly | Thr | Ile | Thr | Val | Leu |
| CGC | GAC | TAC | GGC | ACC | ATC | ACC | GTG | CTG |
| Phe | Asn | Thr | Asp | Glu | Glu | Lys | Gly | Ser |
| TTC | AAC | ACC | GAC | GAG | GAA | AAG | GGT | TCC |
| 180 |  |  |  |  |  |  |  |  |
| Phe | Gly | Ser | Arg | Asp | Leu | Ile | Gln | Glu |
| TTC | GGC | TCG | CGC | GAC | CTG | ATC | CAG | GAA |
|  | 190 |  |  |  |  |  |  |  |
| Glu | Ala | Lys | Leu | Ala | Asp | Tyr | Val | Leu |
| GAA | GCC | AAG | CTG | GCC | GAC | TAC | GTG | CTC |
|  |  | 200 |  |  |  |  |  |  |
| Ser | Phe | Glu | Pro | Thr | Ser | Ala | Gly | Asp |
| TCC | TTC | GAG | CCC | ACC | AGC | GCA | GGC | GAC |
|  |  |  | 210 |  |  |  |  |  |
| Glu | Lys | Leu | Ser | Leu | Gly | Thr | Ser | Gly |
| GAA | AAA | CTC | TCG | CTG | GGC | ACC | TCG | GGC |
|  |  |  |  | 220 |  |  |  |  |
| Ile | Ala | Tyr | Val | Gln | Val | Asn | Ile | Thr |
| ATC | GCC | TAC | GTG | CAG | GTC | AAC | ATC | ACC |
|  |  |  |  |  | 230 |  |  |  |
| Gly | Lys | Ala | Ser | His | Ala | Gly | Ala | Ala |
| GGC | AAG | GCC | TCG | CAT | GCC | GGC | GCC | GCG |

| | | | | | | | | |
|---|---|---|---|---|---|---|---|---|
| Pro | Glu | Leu | Gly | Val | Asn | **240** Ala | Leu | Val |
| CCC | GAG | CTG | GGC | GTG | AAC | GCG | CTG | GTC |
| Glu | Ala | Ser | Asp | Leu | Val | Leu | **250** Arg | Thr |
| GAG | GCT | TCC | GAC | CTC | GTG | CTG | CGC | ACG |
| Met | Asn | Ile | Asp | Asp | Lys | Ala | Lys | **260** Asn |
| ATG | AAC | ATC | GAC | GAC | AAG | GCG | AAG | AAC |
| Leu | Arg | Phe | Asn | Trp | Thr | Ile | Ala | Lys |
| CTG | CGC | TTC | AAC | TGG | ACC | ATC | GCC | AAG |
| **270** Ala | Gly | Asn | Val | Ser | Asn | Ile | Ile | Pro |
| GCC | GGC | AAC | GTC | TCG | AAC | ATC | ATC | CCC |
| Ala | **280** Ser | Ala | Thr | Leu | Asn | Ala | Asp | Val |
| GCC | AGC | GCC | ACG | CTG | AAC | GCC | GAC | GTG |
| Arg | Tyr | **290** Ala | Arg | Asn | Glu | Asp | Phe | Asp |
| CGC | TAC | GCG | CGC | AAC | GAG | GAC | TTC | GAC |
| Ala | Ala | Met | **300** Lys | Thr | Leu | Glu | Glu | Arg |
| GCC | GCC | ATG | AAG | ACG | CTG | GAA | GAG | CGC |
| Ala | Gln | Gln | Lys | **310** Lys | Leu | Pro | Glu | Ala |
| GCG | CAG | CAG | AAG | AAG | CTG | CCC | GAG | GCC |
| Asp | Val | Lys | Val | Ile | **320** Val | Thr | Arg | Gly |
| GAC | GTG | AAG | GTG | ATC | GTC | ACG | CGC | GGC |
| Arg | Pro | Ala | Phe | Asn | Ala | **330** Gly | Glu | Gly |
| CGC | CCG | GCC | TTC | AAT | GCC | GGC | GAA | GGC |
| Gly | Lys | Lys | Leu | Val | Asp | Lys | **340** Ala | Val |
| GGC | AAG | AAG | CTG | GTC | GAC | AAG | GCG | GTG |
| Ala | Tyr | Tyr | Lys | Glu | Ala | Gly | Gly | **350** Thr |
| GCC | TAC | TAC | AAG | GAA | GCC | GGC | GGC | ACG |
| Leu | Gly | Val | Glu | Glu | Arg | Thr | Gly | Gly |
| CTG | GGC | GTG | GAA | GAG | CGC | ACC | GGC | GGC |
| **360** Gly | Thr | Asp | Ala | Ala | Tyr | Ala | Ala | Leu |
| GGC | ACC | GAC | GCG | GCC | TAC | GCC | GCG | CTC |
| Ser | **370** Gly | Lys | Pro | Val | Ile | Glu | Ser | Leu |
| TCA | GGC | AAG | CCA | GTG | ATC | GAG | AGC | CTG |
| Gly | Leu | **380** Pro | Gly | Phe | Gly | Tyr | His | Ser |
| GGC | CTG | CCG | GGC | TTC | GGC | TAC | CAC | AGC |

19

| | | | 390 | | | | | |
|---|---|---|---|---|---|---|---|---|
| Asp | Lys | Ala | Glu | Tyr | Val | Asp | Ile | Ser |
| GAC | AAG | GCC | GAG | TAC | GTG | GAC | ATC | AGC |

| | | | | 400 | | | | |
|---|---|---|---|---|---|---|---|---|
| Ala | Ile | Pro | Arg | Arg | Leu | Tyr | Met | Ala |
| GCG | ATT | CCG | CGC | CGC | CTG | TAC | ATG | GCT |

| | | | | | 410 | | | |
|---|---|---|---|---|---|---|---|---|
| Ala | Arg | Leu | Ile | Met | Asp | Leu | Gly | Ala |
| CGC | CGC | CTG | ATC | ATG | GAT | CTG | GGC | GCC |

| | | | |
|---|---|---|---|
| Gly | Lys | | |
| GGC | AAG | TGA | —3' |

8. Rekombinanter DNA-Transfervektor nach einem der vorstehenden Ansprüche, dadurch gekennzeichnet, daß der Transfervektor ein Plasmid ist.

9. Durch einen Transfervektor transformierter Mikroorganismus, dadurch gekennzeichnet, daß der Transfervektor ein rekombinanter DNA-Transfervektor nach einem der Ansprüche 1 bis 8 ist.

10. Mikroorganismus nach Anspruch 9, der ein Bakterium der Gattung E. coli oder Pseudomonas oder eine Hefe der Gattung Saccharomyces cerevisiae ist.

11. Verfahren zur Herstellung eines Genprodukts, gekennzeichnet durch

(a) Züchten eines Mikroorganismus nach Anspruch 9 oder 10 in einem Kulturmedium zur Herstellung des Genprodukts in Kulturmedium oder im periplasmatischen Raum des Mikroorganismus, und

(b) Isolieren des Genprodukts aus dem Kulturmedium oder dem periplasmatischen Raum des Mikroorganismus.

12. Verfahren nach Anspruch 11, gekennzeichnet durch Herstellung von Pseudomonas-Carboxypeptidase $G_2$ als Genprodukt.

13. Verfahren nach Anspruch 11, gekennzeichnet durch Herstellung eines anderen Proteins oder Polypeptids als Pseudomonas-Carboxypeptidase $G_2$ als Genprodukt.

14. Verfahren nach Anspruch 13, gekennzeichnet durch eines anderen prokaryotischen Proteins als Pseudomonas-Carboxypeptidase $G_2$ als Genprodukt.

15. Verfahren nach Anspruch 14, gekennzeichnet durch β-Galactosidase aus E. coli als Genprodukt.

**Revendications**

1. Un vecteur de transfert d'ADN recombinant comprenant une séquence guide de polynucléotide en aval et en phase de lecture relativement à un promoteur de bactérie ou de levure et à un site de liaison ribosomique procaryotique et en amont et en phase de lecture relativement à un gène de structure, caractérisé en ce que la séquence guide de polynucléotide code pour un polypeptide signal de formule I

Met-Arg-Pro-Ser-Ile-His-Arg-Thr-Ala-Ile-Ala
Ala-Val-Leu-Ala-Thr-Ala-Phe-Val-Ala-Gly-Thr.                    I

2. Un vecteur de transfert d'ADN recombinant selon la revendication 1, caractérisé en ca que la séquence guide de polynucléotide répond à la formule II

| 5'— | ATG | CGC | CCA | TCC | ATC | CAC | CGC | ACA | |
|---|---|---|---|---|---|---|---|---|---|
| | GCC | ATC | GCC | GCC | GTG | CTG | GCC | ACC | II |
| | GCC | TTC | GTG | GCG | GGC | ACC | —3' | | |

3. Un vecteur de transfert d'ADN recombinant selon l'une ou l'autre des revendications 1 ou 2, caractérisé en ce que le gène de structure code pour la carboxypeptidase $G_2$ de Pseudomonas (CPG$_2$).

4. Un vecteur de transfert d'ADN recombinant selon l'une ou l'autre des revendications 1 ou 2, caractérisé en ce que le gène de structure code pour une protéine ou un polypeptide autre que la carboxypeptidase $G_2$ de Pseudomonas.

5. Un vecteur de transfert d'ADN recombinant selon la revendication 4 caractérisé en ce que le gène de structure code pour une protéine procaryotique autre que la carboxypeptidase $G_2$ de Pseudomonas.

6. Un vecteur de transfert d'ADN recombinant selon la revendication 5 caractérisé en ce que le gène de structure code pour la β-galactosidase de E. coli.

7. Un vecteur de transfert d'ADN recombinant selon la revendication 3 comprenant un polynucléotide de formule

|   | **1** |   |   |   |   |   |   |   |
|---|---|---|---|---|---|---|---|---|
| 5'— | Met ATG | Arg CGC | Pro CCA | Ser TCC | Ile ATC | His CAC | Arg CGC | Thr ACA |

|   | **10** |   |   |   |   |   |   |   |
|---|---|---|---|---|---|---|---|---|
| Ala GCC | Ile ATC | Ala GCC | Ala GCC | Val GTG | Leu CTG | Ala GCC | Thr ACC | Ala GCC |

|   |   | **20** |   |   |   |   |   |   |
|---|---|---|---|---|---|---|---|---|
| Phe TTC | Val GTG | Ala GCG | Gly GGC | Thr ACC | Ala GCC | Leu CTG | Ala GCC | Gln CAG |

|   |   |   | **30** |   |   |   |   |   |
|---|---|---|---|---|---|---|---|---|
| Lys AAG | Arg CGC | Asp GAC | Asn AAC | Val GTG | Leu CTG | Phe TTC | Gln CAG | Ala GCA |

|   |   |   |   | **40** |   |   |   |   |
|---|---|---|---|---|---|---|---|---|
| Ala GCT | Thr ACC | Asp GAC | Glu GAG | Gln CAG | Pro CCG | Ala GCC | Val GTG | Ile ATC |

|   |   |   |   |   | **50** |   |   |   |
|---|---|---|---|---|---|---|---|---|
| Lys AAG | Thr ACG | Leu CTG | Glu GAG | Lys AAG | Leu CTG | Val GTC | Asn AAC | Ile ATC |

|   |   |   |   |   |   | **60** |   |   |
|---|---|---|---|---|---|---|---|---|
| Glu GAG | Thr ACC | Gly GGC | Thr ACC | Gly GGT | Asp GAC | Ala GCC | Glu GAG | Gly GGC |

|   |   |   |   |   |   |   | **70** |   |
|---|---|---|---|---|---|---|---|---|
| Ile ATC | Ala GCC | Ala GCT | Ala GCG | Gly GGC | Asn AAC | Phe TTC | Leu CTC | Glu GAG |

|   |   |   |   |   |   |   |   | **80** |
|---|---|---|---|---|---|---|---|---|
| Ala GCC | Glu GAG | Leu CTC | Lys AAG | Asn AAC | Leu CTC | Gly GGC | Phe TTC | Thr ACG |

|   |   |   |   |   |   |   |   |   |
|---|---|---|---|---|---|---|---|---|
| Val GTC | Thr ACG | Arg CGA | Ser AGC | Lys AAG | Ser TCG | Ala GCC | Gly GGC | Leu CTG |

| **90** |   |   |   |   |   |   |   |   |
|---|---|---|---|---|---|---|---|---|
| Val GTG | Val GTG | Gly GGC | Asp GAC | Asn AAC | Ile ATC | Val GTG | Gly GGC | Lys AAG |

|   | **100** |   |   |   |   |   |   |   |
|---|---|---|---|---|---|---|---|---|
| Ile ATC | Lys AAG | Gly GGC | Arg CGC | Gly GGC | Gly GGC | Lys AAG | Asn AAC | Leu CTG |

|   |   | **110** |   |   |   |   |   |   |
|---|---|---|---|---|---|---|---|---|
| Leu CTG | Leu CTG | Met ATG | Ser TCG | His CAC | Met ATG | Asp GAC | Thr ACC | Val GTC |

|   |   |   | **120** |   |   |   |   |   |
|---|---|---|---|---|---|---|---|---|
| Tyr TAC | Leu CTC | Lys AAG | Gly GGC | Ile ATT | Leu CTC | Ala GCG | Lys AAG | Ala GCC |

|   |   |   |   | **130** |   |   |   |   |
|---|---|---|---|---|---|---|---|---|
| Pro CCG | Phe TTC | Arg CGC | Val GTC | Glu GAA | Gly GGC | Asp GAC | Lys AAG | Ala GCC |

|   |   |   |   |   | **140** |   |   |   |
|---|---|---|---|---|---|---|---|---|
| Tyr TAC | Gly GGC | Pro CCG | Gly GGC | Ile ATC | Ala GCC | Asp GAC | Asp GAC | Lys AAG |

| | | | | | | | | |
|---|---|---|---|---|---|---|---|---|
| Gly GGC | Gly GGC | Asn AAC | Ala GCG | Val GTC | Ile ATC | **150** Leu CTG | His CAC | Thr ACG |
| Leu CTC | Lys AAG | Leu CTG | Leu CTG | Lys AAG | Glu GAA | Tyr TAC | **160** Gly GGC | Val GTG |
| Arg CGC | Asp GAC | Tyr TAC | Gly GGC | Thr ACC | Ile ATC | Thr ACC | Val GTG | **170** Leu CTG |
| Phe TTC | Asn AAC | Thr ACC | Asp GAC | Glu GAG | Glu GAA | Lys AAG | Gly GGT | Ser TCC |
| **180** Phe TTC | Gly GGC | Ser TCG | Arg CGC | Asp GAC | Leu CTG | Ile ATC | Gln CAG | Glu GAA |
| Glu GAA | **190** Ala GCC | Lys AAG | Leu CTG | Ala GCC | Asp GAC | Tyr TAC | Val GTG | Leu CTC |
| Ser TCC | Phe TTC | **200** Glu GAG | Pro CCC | Thr ACC | Ser AGC | Ala GCA | Gly GGC | Asp GAC |
| Glu GAA | Lys AAA | Leu CTC | **210** Ser TCG | Leu CTG | Gly GGC | Thr ACC | Ser TCG | Gly GGC |
| Ile ATC | Ala GCC | Tyr TAC | Val GTG | **220** Gln CAG | Val GTC | Asn AAC | Ile ATC | Thr ACC |
| Gly GGC | Lys AAG | Ala GCC | Ser TCG | His CAT | **230** Ala GCC | Gly GGC | Ala GCC | Ala GCG |
| Pro CCC | Glu GAG | Leu CTG | Gly GGC | Val GTG | Asn AAC | **240** Ala GCG | Leu CTG | Val GTC |
| Glu GAG | Ala GCT | Ser TCC | Asp GAC | Leu CTC | Val GTG | Leu CTG | **250** Arg CGC | Thr ACG |
| Met ATG | Asn AAC | Ile ATC | Asp GAC | Asp GAC | Lys AAG | Ala GCG | Lys AAG | **260** Asn AAC |
| Leu CTG | Arg CGC | Phe TTC | Asn AAC | Trp TGG | Thr ACC | Ile ATC | Ala GCC | Lys AAG |
| **270** Ala GCC | Gly GGC | Asn AAC | Val GTC | Ser TCG | Asn AAC | Ile ATC | Ile ATC | Pro CCC |
| Ala GCC | **280** Ser AGC | Ala GCC | Thr ACG | Leu CTG | Asn AAC | Ala GCC | Asp GAC | Val GTG |
| Arg CGC | Tyr TAC | **290** Ala GCG | Arg CGC | Asn AAC | Glu GAG | Asp GAC | Phe TTC | Asp GAC |

| | | | 300 | | | | | |
|---|---|---|---|---|---|---|---|---|
| Ala | Ala | Met | Lys | Thr | Leu | Glu | Glu | Arg |
| GCC | GCC | ATG | AAG | ACG | CTG | GAA | GAG | GGC |
| | | | | 310 | | | | |
| Ala | Gln | Gln | Lys | Lys | Leu | Pro | Glu | Ala |
| GCG | CAG | CAG | AAG | AAG | CTG | CCC | GAG | GCC |
| | | | | | 320 | | | |
| Asp | Val | Lys | Val | Ile | Val | Thr | Arg | Gly |
| GAC | GTG | AAG | GTG | ATC | GTC | ACG | CGC | GGC |
| | | | | | | 330 | | |
| Arg | Pro | Ala | Phe | Asn | Ala | Gly | Glu | Gly |
| CGC | CCG | GCC | TTC | AAT | GCC | GGC | GAA | GGC |
| | | | | | | | 340 | |
| Gly | Lys | Lys | Leu | Val | Asp | Lys | Ala | Val |
| GGC | AAG | AAG | CTG | GTC | GAC | AAG | GCG | GTG |
| | | | | | | | | 350 |
| Ala | Tyr | Tyr | Lys | Glu | Ala | Gly | Gly | Thr |
| GCC | TAC | TAC | AAG | GAA | GCC | GGC | GGC | ACG |
| Leu | Gly | Val | Glu | Glu | Arg | Thr | Gly | Gly |
| CTG | GGC | GTG | GAA | GAG | CGC | ACC | GGC | GGC |
| 360 | | | | | | | | |
| Gly | Thr | Asp | Ala | Ala | Tyr | Ala | Ala | Leu |
| GGC | ACC | GAC | GCG | GCC | TAC | GCC | GCG | CTC |
| | 370 | | | | | | | |
| Ser | Gly | Lys | Pro | Val | Ile | Glu | Ser | Leu |
| TCA | GGC | AAG | CCA | GTG | ATC | GAG | AGC | CTG |
| | | 380 | | | | | | |
| Gly | Leu | Pro | Gly | Phe | Gly | Tyr | His | Ser |
| GGC | CTG | CCG | GGC | TTC | GGC | TAC | CAC | AGC |
| | | | 390 | | | | | |
| Asp | Lys | Ala | Glu | Tyr | Val | Asp | Ile | Ser |
| GAC | AAG | GCC | GAG | TAC | GTG | GAC | ATC | AGC |
| | | | | 400 | | | | |
| Ala | Ile | Pro | Arg | Arg | Leu | Tyr | Met | Ala |
| GCG | ATT | CCG | CGC | CGC | CTG | TAC | ATG | GCT |
| | | | | | 410 | | | |
| Ala | Arg | Leu | Ile | Met | Asp | Leu | Gly | Ala |
| CGC | CGC | CTG | ATC | ATG | GAT | CTG | GGC | GCC |
| Gly | Lys | | | | | | | |
| GGC | AAG | TGA | —3' | | | | | |

8. Un vecteur de transfert d'ADN recombinant selon l'une quelconque des revendications précédentes caractérisé en ce que le vecteur de transfert est un plasmide.

9. Un micro-organisme transformé par un vecteur de transfert caractérisé en ce que le vecteur de transfert est un vecteur de transfert d'ADN recombinant selon l'une quelconque des revendications précédentes 1 à 8.

10. Un micro-organisme selon la revendication 9 qui est une bactérie de l'espèce E. coli ou Pseudomonas ou une levure de l'espèce Saccharomyces cerevisiae.

11. Un procédé pour la préparation d'un produit génique caractérisé par

(a) la culture d'un micro-organisme selon soit la revendication 9 soit la revendication 10 dans un milieu de culture pour produire le produit génique dans le milieu de culture ou dans l'espace périplasmique du micro-organisme et

**0 121 352**

(b) l'isolement du produit génique du milieu de culture ou de l'espace périplasmique du micro-organisme.

12. Un procédé selon la revendication 11 caractérisé en ce que le produit génique est la carboxypeptidase $G_2$ de Pseudomonas.

13. Un procédé selon la revendication 11 caractérisé en ce que le produit génique est une protéine ou un polypeptide autre que la carboxypeptidase $G_2$ de Pseudomonas.

14. Un procédé selon la revendication 13 caractérisé en ce que le produit génique est une protéine procaryotique autre que la carboxypeptidase $G_2$ de Pseudomonas.

15. Un procédé selon la revendication 14 caractérisé en ce que le produit génique est la β-galactosidase de E. coli.

# Fig.1.

RESTRICTION ENZYME MAP OF pNM1

—— REPRESENTS pBR322

RESTRICTION ENZYME MAP OF pNM111

*Fig.2.*

RESTRICTION ENZYME MAP OF pNM14

*Fig.3.*

RESTRICTION ENZYME MAP OF pNM21

*Fig 4.*

RESTRICTION ENZYME MAP OF pNM 22

*Fig.5.*

Sau3A FRAGMENT

(A)

GATCC ———————————— GATC

Bam

—————————————————————————— M13mp7

EcoRI        EcoRI

⬇

(RECREATED DUE TO DOUBLE
BamHI   C IN Sau3A SITE)

————————————————————————— M13
TEMPLATE

EcoRI                    EcoRI

(B)

Ap^r

EcoRI      BamHI      lacZ

————————————————————— pMC1403

Smal

⬇

(C)

Bam          Eco  Bam    lacz

—————————————————————————

Eco          Sma

Fig.6.

pLEC 3
(9·5kb)

EcoRI
Hind III
Xba I
Ava I
EcoR I
EcoR I
Hind III
Bam HI
Sall
Bam HI
Sall
Ava I
Pst I

| | pBR 322 |
| | Yeast 2µ plasmid |
| | Yeast chromosomal leu 2 gene |
| | Pseudomonas carboxypeptidase G2 gene |

*Fig.7*

RESTRICTION ENZYME MAP OF pLEC 3